# EUROPÄISCHE PATENTSCHRIFT

(11) **EP 2 945 600 B1**
(45) Veröffentlichungstag und Bekanntmachung des Hinweises auf die Patenterteilung: **18.10.2017**
(21) Anmeldenummer: 14707314.2
(22) Anmeldetag: 16.01.2014
(51) Int. Cl.: A61K 8/34, A61K 8/49, A61Q 5/00, A61K 8/64, A61Q 19/00, A61K 8/97, A61K 38/00, A61K 8/46, A61Q 7/00, A61K 9/00, A61K 8/20, A61K 31/045, A61K 31/185, A61K 31/198, A61K 31/353, A61K 36/00

(54) **KOPFHAUT-STIMULATIONSPRÄPARATION**
COMPOSITION FOR STIMULATING THE SCALP
COMPOSITION POUR STIMULER LE CUIR CHEVELU

(30) Priorität: 16.01.2013 AT 500262013
(43) Veröffentlichungstag der Anmeldung: 25.11.2015
(73) Patentinhaber: Hairdreams Haarhandels GmbH, 8051 Graz (AT)
(72) Erfinder: OTT, Gerhard, Singapur 118455 (SG)
(74) Vertreter: Patentanwälte Barger, Piso & Partner
(86) Internationale Anmeldenummer: PCT/AT2014/050015
(87) Internationale Veröffentlichungsnummer: WO 2014/110614

(56) Entgegenhaltungen:
- EP-A1- 0 394 920
- EP-A2- 2 238 966
- DE-A1- 3 211 913
- DE-A1-102005 022 626
- JP-A- 2000 038 324
- Lucas Meyer ET AL: "PROVIDING FULLER AND THICKER LOOKING HAIR AnAgen/telogen rAtio", , 1. März 2012 (2012-03-01), XP055113721, Gefunden im Internet: URL:http://www.innovadex.com/documents/112 0281.pdf?bs=4499&b=191081&st=20 [gefunden am 2014-04-11] in der Anmeldung erwähnt
- Anonymous: "GNPD - Hair Serum Booster", , 1. Juni 2012 (2012-06-01), XP055113722, Gefunden im Internet: URL:http://www.gnpd.com/sinatra/recordpage /1813179/from_search/kEps6fXfWL/ [gefunden am 2014-04-11] in der Anmeldung erwähnt
- Anonymous: "Due Penna Hair Care Kit - Buy Hair Care Product on Alibaba.com", , 1. Januar 2012 (2012-01-01), XP055113726, Gefunden im Internet: URL:http://www.alibaba.com/product-detail/ Due-Penna-Hair-Care-Kit_132350510.html [gefunden am 2014-04-11] in der Anmeldung erwähnt

## Beschreibung

Die Erfindung bezieht sich auf eine Kopfhaut-Stimulationspräparation, die Acetyl-Tetrapeptid-3, Biochanin A, Bockshorn-Kleesamenextrakt und Menthol aufweist.

Eine Haarpflege-Behandlungslotion, die außer den vorerwähnten Wirkstoffen noch unzählige Pflanzenextrakte enthält, ist von "Due Penna" auf den Markt gebracht worden. Die Wirksamkeit des Produkts beruht auf dem Synergieeffekt aller Ingredienzien. Besondere Bedeutung kommt dabei dem Acetyl-Tetrapeptid-3 zu, das in Kombination mit Bockshorn-Kleesamenextrakt in flüssigen Haarpflegepräparaten von "HairGro" oder zusammen mit Biochanin A enthaltendem Rotklee-Extrakt in Haarkosmetika von "Lucas Meyer Cosmetics" angeboten wird.

Acetyl-Tetrapeptid-3 stimuliert die Proteinsynthese in der Matrix rund um den Haarfollikel und trägt so zu dessen Festigkeit und besserem Wachstum bei. Es stimuliert die ECM-Proteinbildung im Bereich der dermalen Papillen und hat somit direkten Einfluss auf Größe und Verankerung der Haarfollikel, fördert also das Wachstum von gesundem, dichtem Haar.

Biochanin A wird eine die Hautporengröße verringernde Wirkung zugeschrieben (DE 10 2009 026 718 A1). Zudem wirkt es gegen Haarausfall, indem es die Bildung des Steroidhormons Dihydrotestosteron (DHT) durch Umwandlung des Hormons Testosteron mittels des Enzyms 5a-Reduktase dadurch inhibiert, dass es die Aktivität dieses Enzyms stark reduziert. Der negative Einfluss des DHT auf die dagegen überempfindlichen Haarfollikel und somit die Verkürzung der Wachstumsphase des Haares wird somit gestoppt.

Bockshorn-Kleesamenextrakt ist ein uraltes Naturmittel zum Stärken der Haare und zur Regeneration der Haarwurzeln; schon Hildegard von Bingen hat darüber berichtet. In neuerer Zeit wird in der DE 20 2007 012 586 U1 darauf Bezug genommen. Bockshorn-Kleesamenextrakt wirkt gegen verschiedene Formen des Haarausfalls (zB kreisrunder, hormonbedingter oder medikamentenbedingter Haarausfall) und entfaltet eine Heilwirkung bei dünnem, brüchigem oder schuppigem Haar.

Die Kühl- und Pflegewirkung von Menthol - in Reinform oder als Pflanzenextrakt Mentha piperita aqua. - ist ebenfalls hinlänglich bekannt.

Bei einem Produkt der eingangs genannten Art mit den vorerwähnten Wirkstoffen hat sich herausgestellt, dass seine Wirksamkeit in der Laborpraxis, dh in vitro, wohl den Erwartungen entspricht, bei der tatsächlichen Applikation auf der Kopfhaut, also in vivo, jedoch geringer ist.

Ziel der Erfindung ist die Beseitigung dieses Nachteils, also die Vermeidung einer Degradation von den Haarwuchs fördernden und Haarausfall vermindernden Wirkstoffen.
Dieses Ziel wird im Einklang mit der Erfindung durch eine Kopfhaut-Stimulationspräparation der eingangs genannten Art erreicht, die gekennzeichnet ist durch eine
flüssige Phase, enthaltend in wässriger Lösung (bezogen auf 100 Gew %)
   0,050 bis 0,250 Gew % Acetyl-Tetrapeptid-3
   0,050 bis 0,250 Gew % Biochanin A
   1,500 bis 2,700 Gew % Bockshorn-Kleesamenextrakt
   0,500 bis 1,500 Gew % Menthol,
   sowie eine mit dieser unmittelbar vor der Anwendung vereinigbare
feste Phase, die Kochsalz (z.B. iodiertes Vollsalz) als Trägersubstanz aufweist
   und (bezogen auf 100 Gew %)
   0,10 bis 1,00 Gew % eines Wirkstoffverstärkers für die flüssige Phase, zB Taurin,
enthält, wobei das Gewichtsverhältnis zwischen der flüssigen und der festen Phase ungefähr 50 zu 1 beträgt.

Die flüssige Phase der erfindungsgemäßen Präparation enthält an sich (jeweils einzeln oder in Kombination) bekannte Wirkstoffe in speziellen Mengenverhältnissen. Die feste Phase, die als Trägersubstanz Salz, zB jodiertes Vollsalz, aufweist und einen Wirkstoffverstärker enthält, wirkt insgesamt verstärkend auf die in der flüssigen Phase enthaltenen Wirkstoffe. Die sanfte Heilkraft und katalytische Wirkung von Kochsalz (Natriumchlorid mit Spuren von Kaliumchlorid, Jod uam) ist seit langem bekannt (Meerbäder, kontinentale Kurbäder). Thermen und Kuranstalten nützen den Effekt solehaltiger Wirkstoffkatalysatoren aus. Körperwarme Salzlösungen regen die Durchblutung und den Stoffwechsel der Haut an. Wirkstoffverstärker als solche sind in der Fachwelt allgemein bekannt. Darunter fallen zB Coffeine, Taurin, Glycose-Dextron-Verbindungen uam. Der Wirkungsmechanismus von Taurin wurde zB in der DE 10 2005 022 626 A1 beschrieben: Taurin stimuliert die Synthese von epidermalen Barrierelipiden und inhibiert cytotoxische sowie proinflammatorische Vorgänge. Dadurch, dass die beiden Phasen erst im Zuge der Präparataufbringung auf die Kopfhaut vereinigt werden, erfolgt der Verstärkungseffekt genau im richtigen Moment; andernfalls, dh wären Salz und der Wirkstoffverstärker bereits a priori in der Tinktur enthalten, würde dieser Boostereffekt verloren gehen. Die getrennte Aufbewahrung beider Phasen kann in ein und demselben Gebinde erfolgen, zB in einem Fläschchen, in dessen Flaschenkörper die flüssige Phase, in dessen Deckel aber die feste Phase untergebracht ist, sodass die beiden Phasen beim Ausgießen vereinigt werden.

Um die Stimulation des Haarwuchses weiter zu begünstigen und den Haarausfall zu verringern, hat es sich als vorteilhaft erwiesen, der flüssigen Phase der erfindungsgemäßen Kopfhaut-Stimulationspräparation noch 0,003 bis 0,045 Gew % L-Aminosäuren beizumengen. Diese - L-Cystein, L-Glutamin und L-Methionin - sind bekanntermaßen Eiweißstoffe und wesentliche Bestandteile von Haut und Haaren. In Cremen, Lotionen odgl wirken sie feuchtigkeitsbewahrend sowie haut- und haarglättend.

Zwecksmäßigerweise kann der flüssigen Phase der erfindungsgemäßen Kopfhaut-Stimulationspräparation 0,01 bis 0,60 Gew % eines UV-Schutzmittels, beispielsweise Glykoläther, beigemengt werden. Dies trägt zur generellen Bewahrung gesunder Kopfhaut bei und unterstützt so den Haarwuchs, wogegen ein Haarausfall weiter inhibiert wird.

Weiters hat sich gezeigt, dass durch Zusatz von 5,0 bis 15,0 Gew % leicht alkalischem Thermalwasser zu der flüssigen Phase der Präparation die Kopfhautflora zusätzlich positiv stimuliert werden kann. Diese Thermalwässer haben einen pH-Wert von etwa 7,05 bis 7,50 und enthalten entweder zB gelöste Schwefelwasserstoffe in einer Konzentration von etwa 0,5 bis 25 mg/l und - neben weiteren Inhaltsstoffen wie dreiwertiges Eisen - zweiwertigen Schwefel in einer Menge von etwa 1,8 bis 18,5 mg/l. Die positive Wirkung von Thermalwasser bei der Behandlung und Heilung von dermatologischen Erkrankungen und seine keratolytischen Effekte sind hinlänglich bekannt.
Von Vorteil ist, wenn der festen Phase noch 0,01 bis 0,10 Gew % wenigstens einer der Aminosäuren L-Cystein, L-Glutamin, L-Methionin zugesetzt werden, um so die in der flüssigen Phase bereits enthaltenen L-Aminosäuren in ihrer kopfhautnährenden und haarkonditionierenden Wirkung zu unterstützen.

Schließlich ist es auch günstig, der festen Phase 0,1 bis 1,0 Gew % an Mentholkristallen beizumengen, um den Kühleffekt des in der flüssigen Phase bereits enthaltenen Menthols noch zu verstärken.
Die Erfindung wird anhand einer beispielsweisen Rezeptur der dominierenden Bestandteile der Kopfhaut-Stimulationspräparation näher erläutert.

| Flüssige Phase | Gew % |
|---|---|
| Wasser (gereinigt, entsalzt) | 61,86 |
| Äthanol (vergällt) | 20,00 |
| Acetyl-Tetrapeptid-3 | 0,250 |
| Biochanin A | 0,250 |
| Bockshorn- Kleesamenextrakt | 2,475 |
| Menthol | 1,500 |

optional
Thermalwasser
(pH-Wert: 7,15
gelöste Schwefelwasserstoffe 24,9 mg/l

| | |
|---|---|
| zweiwertiger Schwefel 18,5 mg/l) | 5,000 |
| L-Aminosäuren | 0,045 |
| Glykoläther | 0,500 |
| Pflanzenextrakte | 0,180 |
| Natriumphosphat | 1,100 |
| Zitronensäure | 0,720 |
| Ätherische Öle | 0,100 |

**Feste Phase**

| | |
|---|---|
| Salz (Vollsalz, jodiert) | 97,70 |
| Taurin | 1,00 |

optional

**L-Aminosäuren**

| | |
|---|---|
| (L-Cystein, L-Glutamin, L-Methionin) | 0,100 |
| Menthol | 1,000 |

### Gewichtsverhältnis flüssig : fest = 50 : 1

Die gegenständliche Zweiphasen-Präparation wird in an sich bekannten Deckelbehälter-Fläschchen aufbewahrt - die flüssige Phase im Flaschenkörper, die feste im Deckelbehälter. Die Vereinigung der beiden Phasen erfolgt somit erst kurz vor der Anwendung der Präparation - ansonsten würde die katalytische Wirkung der festen Phase infolge verfrühter Zugabe verpuffen. Die Applikation des Präparates erfolgt durch Einmassieren in die Kopfhaut - bei bereits eingetretenen Kopfhaut- oder Haarproblemen einmal täglich während einiger Monate; zur vorbeugenden Pflege etwa einmal pro Woche. Beste Wirkungen haben sich bis jetzt gezeigt, wenn die gegenständliche Kopfhaut-Stimulationspräparation in unmittelbarem Anschluss an eine Behandlung mit einer Kopfhaut-Pflegepräparation derselben Herstellerin angewandt wird, da diesfalls einerseits ein weiterer Synergieeffekt der pflanzlichen Wirkstoffe eintritt, also Phytotherapie zur Geltung kommt, anderseits durch die Kühlwirkung des Menthols die physikalische Stimulanz von Wärme und Kälte, also Hydrotherapie, ausgenützt wird. Die Boosterwirkung des in der festen Phase der Präparation enthaltenen Wirkstoffverstärkers, zB Taurin, die bei Vereinigung mit der flüssigen Phase eintritt, war bei den bislang durchgeführten Kurzzeitstudien beeindruckend; im Vergleich zu unbehandelten bzw placebobehandelten Personen stieg die Haardichte merklich. Langzeitstudien sind erst im Gange.

## Patentansprüche

1. Kopfhaut-Stimulationspräparation, die Acetyl-Tetrapeptid-3, Biochanin A, Boxhorn-Kleesamenextrakt und Menthol aufweist, **gekennzeichnet durch** eine
flüssige Phase, enthaltend in wässriger Lösung (bezogen auf 100 Gew %)
0,050 bis 0,250 Gew % Acetyl-Tetrapeptid-3
0,050 bis 0,250 Gew % Biochanin A
1,500 bis 2,700 Gew % Bockshorn-Kleesamenextrakt
0,500 bis 1,500 Gew % Menthol,
sowie eine mit dieser unmittelbar vor der Anwendung vereinigbare
feste Phase, die Kochsalz, zB jodiertes Vollsalz als Trägersubstanz aufweist und (bezogen auf 100 Gew %)
0,10 bis 1,00 Gew % eines Wirkstoffverstärkers für die flüssige Phase, zB Taurin,
enthält, wobei das Gewichtsverhältnis zwischen der flüssigen und der festen Phase ungefähr 50 zu 1 beträgt.

2. Kopfhaut-Stimulationspräparation nach Anspruch 1, **dadurch gekennzeichnet, dass** der flüssigen Phase 0,003 bis 0,045 Gew % L-Aminosäuren beigemengt sind.

3. Kopfhaut-Stimulationspräparation nach Anspruch 1 oder 2, **dadurch gekennzeichnet, dass** der flüssigen Phase 0,01 bis 0,60 Gew % eines UV-Schutzmittels, beispielsweise Glykoläther, beigemengt sind.

4. Kopfhaut-Stimulationspräparation nach einem der Ansprüche 1 bis 3, **dadurch gekennzeichnet, dass** der flüssigen Phase 5,0 bis 15,0 Gew % leicht alkalisches Thermalwasser zugesetzt sind.

5. Kopfhaut-Stimulationspräparation nach einem der Ansprüchen 1 bis 4, **dadurch gekennzeichnet, dass** der festen Phase 0,01 bis 0,10 Gew % wenigstens einer der Aminosäuren L-Cystein, L-Glutamin, L-Methionin zugesetzt sind.

6. Kopfhaut-Stimulationspräparation nach einem der Ansprüche 1 bis 5, **dadurch gekennzeichnet, dass** der festen Phase 0,1 bis 1,0 Gew % an Mentholkristallen beigemengt sind.

## Claims

1. Scalp-Stimulation Preparation comprising Acetyl-Tetrapeptide-3, Biochanin A, Fenugreek Seed Extract and Menthol, **characterized**
**by** a liquid phase containing in aqueous solution (related to 100 weight %)
0,050 to 0,250 weight % Acetyl-Tetrapeptide-3
0,050 to 0,250 weight % Biochanin A
1,500 to 2,700 weight % Fenugreek Seed Extract
0,500 to 1,500 weight % Menthol,
and by a solid phase, to be united with it immediately before the application, which comprises table salt, e.g. iodized salt, as carrier substance and (related to 100 weight %) contains
0,10 to 1,00 weight % of an active-substance-booster for the liquid phase, e.g. Taurine,
whereby the weight relation between the liquid and the solid phase is approximately 50 to 1.

2. Scalp-Stimulation-Preparation according to claim 1, **characterized in that** 0,003 to 0,045 weight % of L-Amino acid is mixed into the liquid phase.

3. Scalp-Stimulation-Preparation according to claim 1 or 2, **characterized in that** 0,01 to 0,60 weight % of a UV-protector, e.g. glycol ether, is mixed into the liquid phase.

4. Scalp-Stimulation-Preparation according to one of the claims 1 to 3, **characterized in that** 5,0 to 15,0 weight % of a slightly alkaline thermal water are added to the liquid phase.

5. Scalp-Stimulation-Preparation according to one of the claims 1 to 4, **characterized in that** 0,01 to 0,10 weight % of at least one of the amino acids L-cysteine, L-glutamine, L-methionine are added to the solid phase.

6. Scalp-Stimulation-Preparation according to one of the claims 1 to 5, **characterized in that** 0,01 to 1,0 weight % of menthol crystals are mixed into the solid phase.

## Revendications

1. Préparation de stimulation du cuir chevelu, qui présente de l'acétyl-tétrapeptide-3, de la biochanine A, de l'extrait de graines de fenugrec et du menthol, **caractérisée par** une phase liquide, contenant, en solution aqueuse (par rapport à 100% en poids)
0,050 à 0,250% en poids d'acétyl-tétrapeptide-3
0,050 à 0,250% en poids de biochanine A
1,500 à 2,700% en poids d'extrait de graines de fenugrec
0,500 à 1,500% en poids de menthol,
ainsi que par une phase solide à réunir avec celle-ci juste avant l'application, qui présente du chlorure de sodium, par exemple du chlorure de sodium iodé, comme substance support et (par rapport à 100% en poids)
0,10 à 1,00% en poids d'un agent de renforcement de la substance active pour la phase liquide, par exemple de la taurine,
le rapport pondéral entre la phase liquide et la phase solide étant d'environ 50:1.

2. Préparation de stimulation du cuir chevelu selon la revendication 1, **caractérisée en ce que** 0,003 à 0,045% en poids d'acides L-aminés est mélangé à la phase liquide.

3. Préparation de stimulation du cuir chevelu selon la revendication 1 ou 2, **caractérisée en ce que** 0,01 à 0,06% en poids d'un agent de protection contre les UV, par exemple du glycoléther, est mélangé à la phase liquide.

4. Préparation de stimulation du cuir chevelu selon la revendication 1 à 3, **caractérisée en ce que** 5,0 à 15,0% en poids d'eau thermale légèrement alcaline sont ajoutés à la phase liquide.

5. Préparation de stimulation du cuir chevelu selon l'une quelconque des revendications 1 à 4, **caractérisée en ce que** 0,01 à 0,10% en poids d'au moins un des acides aminés L-cystéine, L-glutamine, L-méthionine est ajouté à la phase solide.

6. Préparation de stimulation du cuir chevelu selon la revendication 1 à 5, **caractérisée en ce que** 0,1 à 1,0% en poids de cristaux de menthol est mélangé à la phase solide.
